# EUROPEAN PATENT APPLICATION

(11) **EP 4 249 111 A1**
(43) Date of publication of application: **27.09.2023**
(21) Application number: 21919468.5
(22) Date of filing: 26.05.2021
(51) Int. Cl.: B01J 19/12, B41J 2/01

(54) **ACTIVE ENERGY IRRADIATION DEVICE AND ACTIVE ENERGY IRRADIATION SYSTEM**

(30) Priority: 13.01.2021 JP 2021003348
(71) Applicant: Hamamatsu Photonics K.K., Hamamatsu-shi, Shizuoka 435-8558 (JP)
(72) Inventor: MURAYAMA Kyoichi, Hamamatsu-shi, Shizuoka 435-8558 (JP); UMENO Keita, Hamamatsu-shi, Shizuoka 435-8558 (JP)
(74) Representative: Grünecker Patent- und Rechtsanwälte PartG mbB
(86) International application number: PCT/JP2021/020025
(87) International publication number: WO 2022/153575

(57) **Abstract**

An active energy irradiation device includes: an active energy irradiation unit having an emitting surface configured to emit an active energy ray; and an inert gas supply unit having a spray port configured to spray an inert gas. The inert gas supply unit includes a housing provided with the spray port, a connection portion provided to the housing, and a throttle portion provided to the connection portion. A pipe for supplying the inert gas into the housing is connectable to the connection portion.

## Description

### Technical Field

The present disclosure relates to an active energy irradiation device and an active energy irradiation system.

### Background Art

An active energy irradiation device has been known that includes an active energy irradiation unit that irradiates an irradiation target with active energy rays, and an inert gas supply unit that supplies an inert gas to a region between the irradiation target and the active energy irradiation unit (for example, refer to Patent Literature 1). In such an active energy irradiation device, in order to prevent oxygen in the air from inhibiting an effect that occurs in the irradiation target due to irradiation with active energy rays, the inert gas is supplied to the region between the irradiation target and the active energy irradiation unit.

### Citation List

### Patent Literature

Patent Literature 1: International Publication WO 2017/170949

### Summary of Invention

### Technical Problem

In the supply unit (inert gas supply unit) of the light irradiation device (active energy irradiation device) described in Patent Literature 1, a porous portion is provided at an end portion (end portion facing an object (irradiation target)) of a space portion into which the inert gas is introduced, such that the inert gas is uniformly sprayed to the object. However, since the pressure of the inert gas inside the space portion increases, the supply unit of the light irradiation device described in Patent Literature 1 is subjected to structural constraints such as the need to improve the pressure resistance and airtightness of the space portion.

An object of the present disclosure is to provide an active energy irradiation device and an active energy irradiation system that are less susceptible to structural constraints, and that can uniformly spray an inert gas to an irradiation target.

### Solution to Problem

According to one aspect of the present disclosure, there is provided an active energy irradiation device including: an active energy irradiation unit having an emitting surface configured to emit an active energy ray; and an inert gas supply unit having a spray port configured to spray an inert gas. The inert gas supply unit includes a housing provided with the spray port, a connection portion provided to the housing, and a throttle portion provided to the connection portion. A pipe for supplying the inert gas into the housing is connectable to the connection portion.

In this active energy irradiation device, the connection portion is provided to the housing provided with the spray port, and the throttle portion is provided to the connection portion. Accordingly, for example, even when the pressure of the inert gas fluctuates within the pipe connected to the connection portion, since the inert gas flowing from the pipe into the housing through the connection portion passes through the throttle portion, the flow rate of the inert gas supplied into the housing is made uniform, and as a result, the flow rate of the inert gas sprayed from the spray port is also made uniform. In addition, since the pressure of the inert gas decreases inside the housing, there is also no need to improve the pressure resistance and airtightness of the housing. Therefore, this active energy irradiation device is less susceptible to structural constraints, and can uniformly spray the inert gas to an irradiation target.

In the active energy irradiation device according to one aspect of the present disclosure, the emitting surface may extend in both a first direction and a second direction perpendicular to the first direction, and be configured to emit the active energy ray to one side in a third direction perpendicular to both the first direction and the second direction, and the spray port may be located on one side in the second direction with respect to the emitting surface, and is configured to spray the inert gas to the one side in the third direction. Accordingly, when the irradiation target is conveyed from the one side in the second direction to the other side in the second direction with respect to the emitting surface of the active energy irradiation unit, the inert gas sprayed from the spray port of the inert gas supply unit is supplied to a region between the irradiation target and the active energy irradiation unit. Therefore, the mixing of oxygen into the region between the irradiation target and the active energy irradiation unit can be effectively suppressed.

In the active energy irradiation device according to one aspect of the present disclosure, the spray port may have an elongated shape with the first direction as a longitudinal direction. Accordingly, the inert gas can be uniformly sprayed to the irradiation target conveyed from the one side in the second direction to the other side in the second direction with respect to the emitting surface of the active energy irradiation unit.

In the active energy irradiation device according to one aspect of the present disclosure, the throttle portion may include a porous filter. In this case, with a simple structure, the flow rate of the inert gas supplied into the housing can be made uniform.

In the active energy irradiation device according to one aspect of the present disclosure, the porous filter may be made of a metal sintered body. In this case, with a simple structure, the flow rate of the inert gas supplied into the housing can be made uniform.

In the active energy irradiation device according to one aspect of the present disclosure, the throttle portion may be provided to the connection portion so as to be located inside the housing. Alternatively, in the active energy irradiation device according to one aspect of the present disclosure, the throttle portion may be provided to the connection portion so as to be located outside the housing. Even in both cases, the flow rate of the inert gas supplied into the housing can be made uniform.

In the active energy irradiation device according to one aspect of the present disclosure, the throttle portion may have a resistance causing a pressure of the inert gas within the pipe to be 0.1 MPa or higher, the pipe connected to the connection portion. Accordingly, the flow rate of the inert gas supplied into the housing can be effectively made uniform.

In the active energy irradiation device according to one aspect of the present disclosure, the throttle portion may be detachably attached to the connection portion. Accordingly, the replacement and maintenance of the throttle portion can be easily performed.

In the active energy irradiation device according to one aspect of the present disclosure, the active energy irradiation unit may emit an ultraviolet ray or an electron beam as the active energy ray. Accordingly, the active energy irradiation device can be used as a device that irradiates the irradiation target with an ultraviolet ray or an electron beam.

According to one aspect of the present disclosure, there is provided an active energy irradiation system including: a plurality of active energy irradiation devices each of which is the active energy irradiation device described above; a first pipe for supplying the inert gas into the housing of each of the plurality of active energy irradiation devices; and a plurality of second pipes branching off from the first pipe, and connected to the connection portion of each of the plurality of active energy irradiation devices. The plurality of active energy irradiation devices are arranged in at least one row.

According to the active energy irradiation system, the flow rate of the inert gas sprayed from each of a plurality of the inert gas supply units can be made uniform in a direction in which the plurality of active energy irradiation devices are arranged.

### Advantageous Effects of Invention

According to the present disclosure, it is possible to provide the active energy irradiation device and the active energy irradiation system that are less susceptible to structural constraints, and that can uniformly spray the inert gas to the irradiation target.

### Brief Description of Drawings

FIG. 1 is a perspective view of an active energy irradiation system according to one embodiment.
FIG. 2 is a perspective view of an active energy irradiation device illustrated in FIG. 1.
FIG. 3 is a perspective view of the active energy irradiation device illustrated in FIG. 2 when viewed from below.
FIG. 4 is an exploded perspective view of the active energy irradiation device illustrated in FIG. 2.
FIG. 5 is a perspective view illustrating an internal configuration of a housing in the active energy irradiation device illustrated in FIG. 2.
FIG. 6 is a front view illustrating the flows of air in the active energy irradiation device illustrated in FIG. 2.
FIG. 7 is a cross-sectional view of the active energy irradiation device taken along line A-A illustrated in FIG. 6.
FIG. 8 is an end view of the active energy irradiation device taken along line B-B illustrated in FIG. 7.
FIG. 9 is a cross-sectional view of an inert gas supply unit of the active energy irradiation device illustrated in FIG. 1.
FIG. 10 is a partial cross-sectional view of the active energy irradiation device illustrated in FIG. 1.
FIG. 11 is a front view of active energy irradiation systems according to a comparative example and an example.
FIG. 12 is a front view of active energy irradiation systems according to a comparative example and an example.

### Description of Embodiments

Hereinafter, an embodiment of the present disclosure will be described in detail with reference to the drawings. Incidentally, in the drawings, the same or equivalent portions are denoted by the same reference signs, and a duplicated description will be omitted.

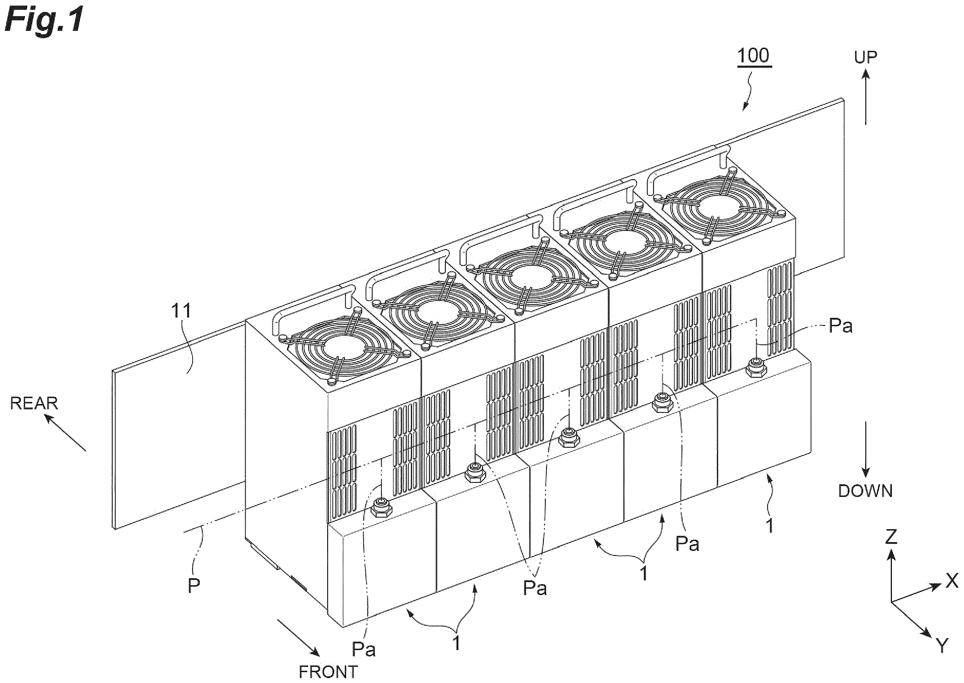

As illustrated in FIG. 1, an active energy irradiation system 100 is, for example, a system that is installed in an ultraviolet (UV) printer, and includes a plurality of active energy irradiation devices 1; a first pipe P for supplying an inert gas to each of the active energy irradiation devices 1; and a plurality of second pipes (pipes) Pa that branch off from the first pipe P, and that are each connected to the active energy irradiation devices 1. The plurality of active energy irradiation devices 1 have the same configuration. The active energy irradiation devices 1 are, for example, high-output air-cooled LED light sources for printing applications. The active energy irradiation devices 1 irradiate an irradiation target with ultraviolet rays (active energy rays) to dry ink on the irradiation target. Examples of the irradiation target include a printed matter to which a photocurable ink is adhered. Incidentally, in FIG. 1, the first pipe P and the plurality of second pipes Pa are illustrated by alternate long and two short dashed lines.

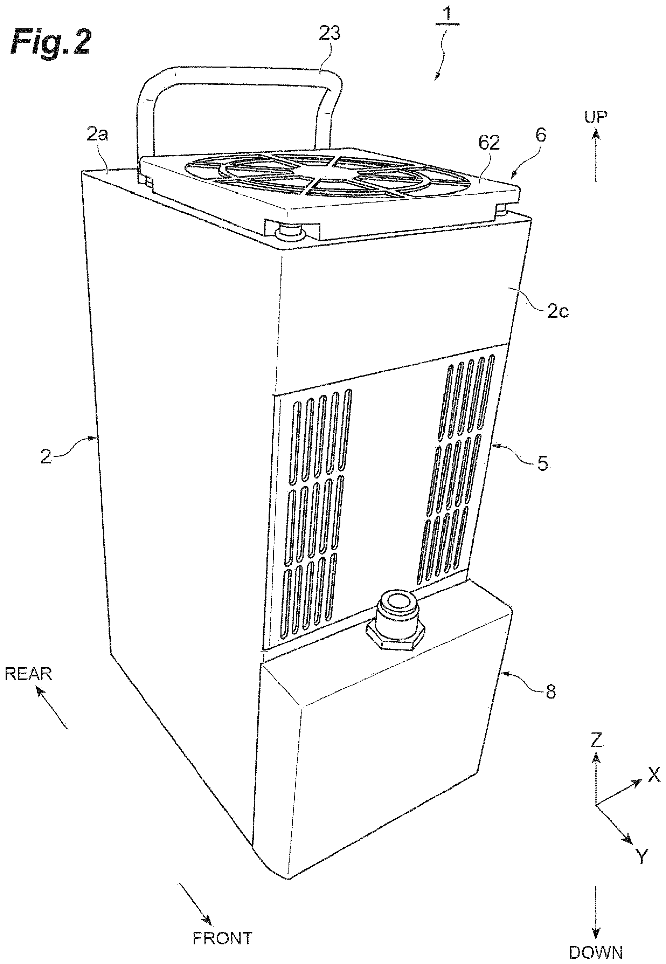

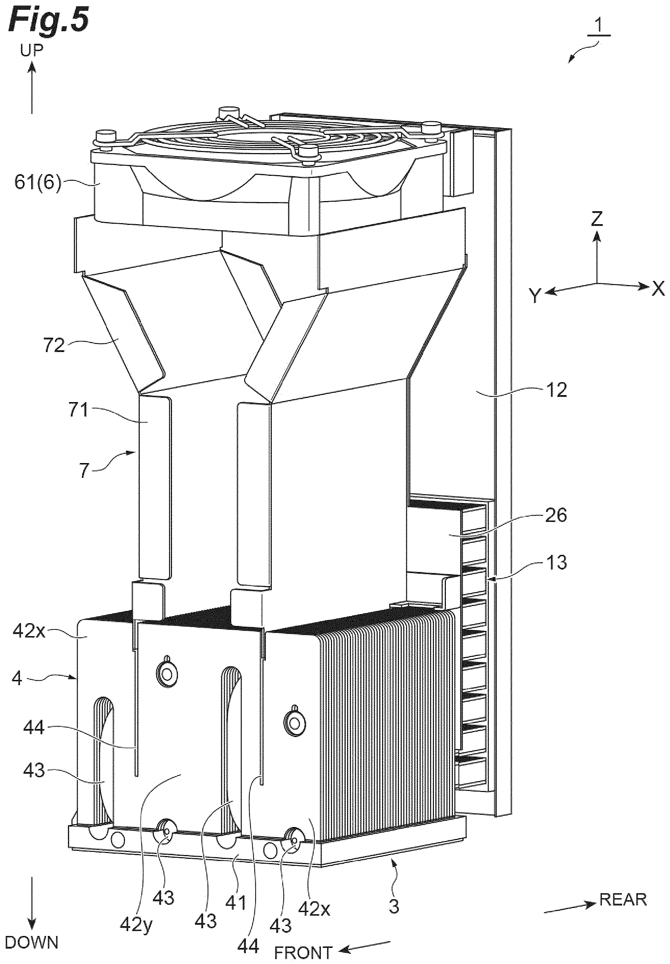

The active energy irradiation devices 1 have a rectangular parallelepiped outer shape. The plurality of active energy irradiation devices 1 are arranged in at least one row. Specifically, the plurality of active energy irradiation devices 1 are arranged in one row so as to be in contact with each other in a predetermined direction. The plurality of active energy irradiation devices 1 arranged in the predetermined direction are fixed and held by a fixing plate 11. In the illustrated example, the active energy irradiation system 100 includes a unit including the plurality of active energy irradiation devices 1 fixed to the fixing plate 11. As illustrated in FIGS. 2, 3, 4, and 5, the active energy irradiation device 1 includes a housing 2, an active energy irradiation unit 3, a heatsink 4, an intake unit 5, an exhaust unit 6, a duct 7, an inert gas supply unit 8, and an inert gas suction unit 9.

Incidentally, for convenience of description, the predetermined direction in which the plurality of active energy irradiation devices 1 are arranged is defined as an "X direction" (first direction), a direction perpendicular to the X direction, which is an emission direction of ultraviolet rays from the active energy irradiation devices 1, is defined as a "Z direction" (third direction perpendicular to both the first direction and a second direction), and a direction orthogonal to the X direction and the Z direction is defined as a "Y direction" (second direction perpendicular to the first direction). A side of the active energy irradiation device 1 from which ultraviolet rays are emitted is defined as a "lower side" (one side in the Z direction), and the opposite side is defined as an "upper side". One side in the Y direction is defined as a "front side", and the other side in the Y direction is defined as a "rear side".

The housing 2 has a rectangular shape that is elongated in the Z direction. The housing 2 is made of metal. The housing 2 houses and supports the active energy irradiation unit 3, the heatsink 4, and the duct 7. The housing 2 is configured by assembling a front casing 21 and a rear casing 22 to each other. A grip portion 23 for grasping the housing 2 is provided on an upper wall 2a of the housing 2. A driver substrate 12 with the Y direction as a thickness direction is disposed on the rear side inside the housing 2. The driver substrate 12 is an electrical driving circuit substrate for driving the active energy irradiation device 1. A driver substrate heatsink 13 that cools transistors and the like of the driver substrate 12 is disposed on the driver substrate 12. The driver substrate heatsink 13 is thermally connected to the transistors and the like of the driver substrate 12.

The active energy irradiation unit 3 includes a substrate 31 having a rectangular plate shape and constituting a predetermined circuit, and LED elements 32 that are light-emitting elements arranged at predetermined pitches in the X direction and the Y direction on the substrate 31. The LED elements 32 emit ultraviolet rays downward. The active energy irradiation unit 3 is disposed at a lower end portion inside the housing 2 with the Z direction as a thickness direction of the substrate 31. A plurality of the substrates 31 are arranged in the X direction. Accordingly, several to several hundred LED elements 32 are arranged in the X direction and the Y direction inside the housing 2. The irradiation target moving in the Y direction is irradiated with ultraviolet rays emitted from each of the LED elements 32 of the active energy irradiation unit 3 through a light irradiation window 24 made of a glass plate and provided on a lower wall 2b of the housing 2. Incidentally, the plurality of substrates 31 may be arranged in the X direction and the Y direction.

The heatsink 4 is a heat radiation member thermally connected to the LED elements 32 of the active energy irradiation unit 3. The heatsink 4 is an air-cooled heatsink that radiates heat through heat exchange with air. Air constitutes a heat transfer medium (coolant) for cooling the LED elements 32. The heatsink 4 includes a base plate 41, heat radiation fins 42, a heat pipe 43, and a partition plate (partition member) 44.

The base plate 41 has a rectangular plate shape. The active energy irradiation unit 3 is provided on a lower surface of the base plate 41. The lower surface of the base plate 41 is in contact with the substrate 31 of the active energy irradiation unit 3. The heat radiation fins 42 have a flat plate shape with the Y direction as a thickness direction. The heat radiation fins 42 are erected on an upper surface (surface) of the base plate 41. The heat radiation fins 42 are arranged to be stacked with gaps therebetween in the Y direction.

The heat pipe 43 is provided to be embedded in a plurality of the heat radiation fins 42. The heat pipe 43 is thermally connected to the plurality of heat radiation fins 42. The partition plate 44 is provided to intersect the plurality of heat radiation fins 42. The partition plate 44 has a flat plate shape with the X direction as a thickness direction. The partition plate 44 partitions the plurality of heat radiation fins 42 in the X direction. A pair of the partition plates 44 are provided spaced from each other in the X direction on the plurality of heat radiation fins 42. The pair of partition plates 44 partition the plurality of heat radiation fins 42 into a pair of outer portions 42x located outside in the X direction and an inner portion 42y located between the pair of outer portions.

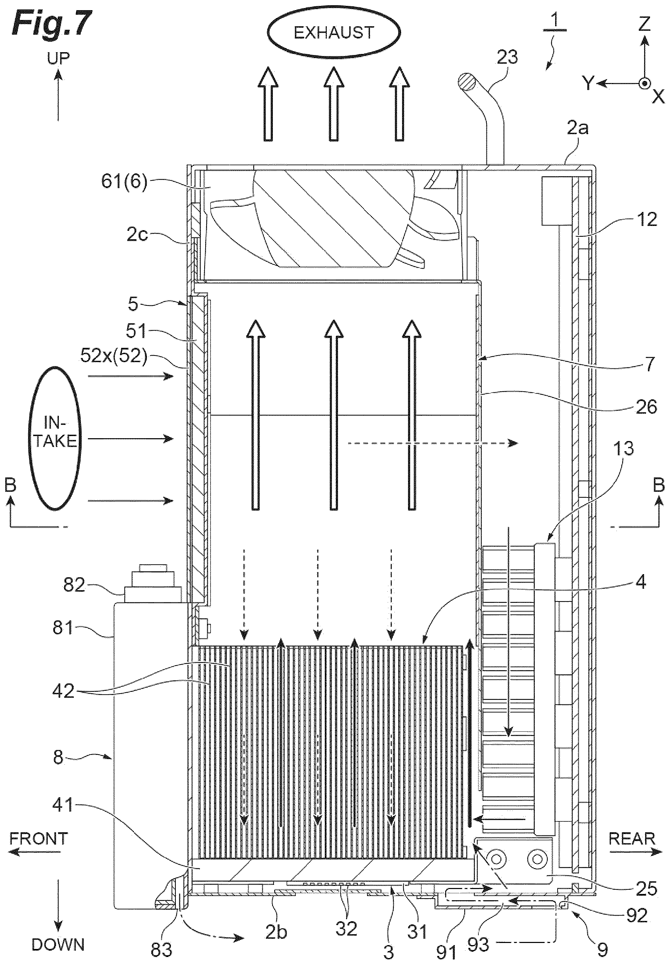

Ends on a base plate 41 side of the partition plates 44 are spaced apart from the base plate 41. Namely, the partition plates 44 partition the plurality of heat radiation fins 42 such that more air passes through in the X direction between the plurality of heat radiation fins 42 on the lower side (base plate 41 side) than on the upper side (side opposite to the base plate 41 side). The partition plates 44 are brazed and fixed to the plurality of heat radiation fins 42. The heatsink 4 is attached to the housing 2 through a bracket 25 and a support frame 26 (refer to FIG. 7).

The intake unit 5 introduces air from outside the housing 2 into the housing 2. The intake unit 5 introduces the air into a buffer space BF to be described later inside the housing 2. The intake unit 5 is provided on a portion toward an upper side of the center of a wall portion 2c on the front side of the housing 2. The intake unit 5 includes an intake filter (filter unit) 51, a filter holding portion 52, and intake ports 53.

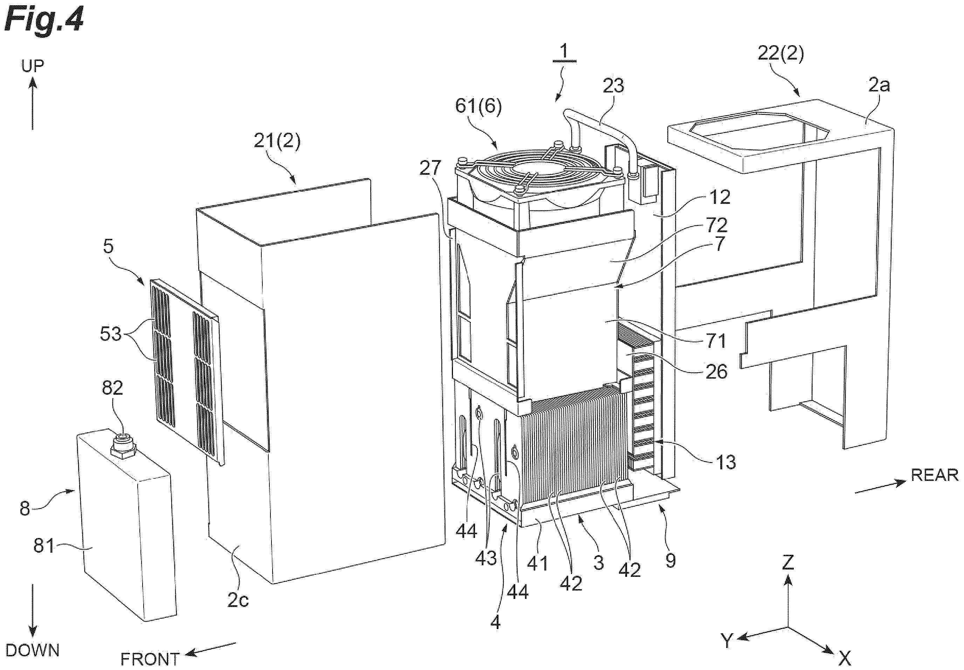

As illustrated in FIGS. 4, 5, 6, and 7, the intake filter 51 captures foreign matter (dust and the like) contained in the air introduced into the housing 2. The intake filter 51 is made of, for example, urethane or the like. The intake filter 51 has a rectangular plate-shaped outer shape. The intake filter 51 extends over the portion toward the upper side of the center of the wall portion 2c when viewed from the front. The filter holding portion 52 houses and holds the intake filter 51. The filter holding portion 52 includes an outer plate 52x having a rectangular plate shape with the Y direction as a thickness direction. A front surface of the outer plate 52x is located on the same plane as a front surface of the wall portion 2c of the housing 2. The filter holding portion 52 is detachably attached to the duct 7 and the support frame 27 provided on the duct 7.

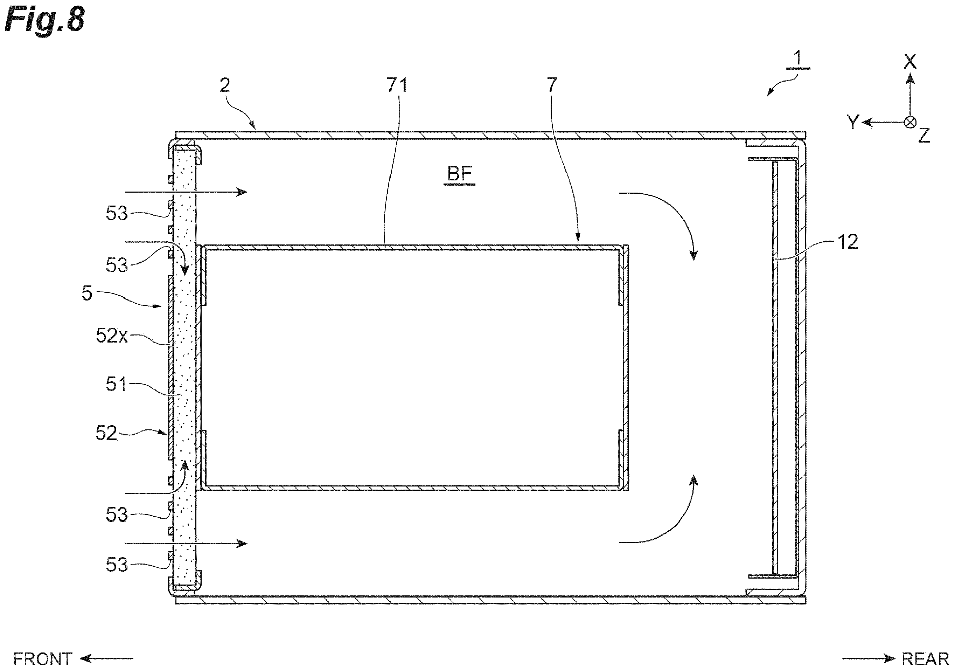

The intake ports 53 are through-holes that are open along the Y direction (direction intersecting a direction from the heatsink 4 toward the exhaust unit 6), and that communicate with the inside of the housing 2. The intake ports 53 are arranged in proximity to each other in regions at both end portions in the X direction of the outer plate 52x. The intake ports 53 are through-holes having an elongated hole shape with the Z direction as a longitudinal direction. Air suctioned from the intake ports 53 is introduced into the buffer space BF inside the housing 2 through the intake filter 51 (refer to FIG. 8).

The exhaust unit 6 discharges the air from inside the housing 2 to the outside of the housing 2. The exhaust unit 6 is provided on an upper end portion of the housing 2. The exhaust unit 6 includes a fan 61. For example, an axial fan is used as the fan 61. The fan 61 delivers the air, which is suctioned from the lower side along the Z direction, to the upper side under pressure along the Z direction. The fan 61 is fixed to an upper end portion inside the housing 2. An exhaust filter 62 made of, for example, urethane or the like is attached to the upper wall 2a of the housing 2 located on a discharge side of the fan 61. Incidentally, the exhaust filter 62 is illustrated only in the FIG. 2 for the sake of convenience, and the illustration in the other drawings is omitted. For example, an external pipe for outdoor exhaust (not illustrated) is connected to the discharge side of the fan 61 of the exhaust unit 6.

The duct 7 is provided between the heatsink 4 and the exhaust unit 6 inside the housing 2. The duct 7 allows the air, which has passed through the heatsink 4, to flow through to the exhaust unit 6. The duct 7 allows an inert gas, which has passed through the heatsink 4, to flow through to the exhaust unit 6. The duct 7 has a rectangular pipe shape. The duct 7 includes a linear portion 71 extending in the Z direction with a constant cross-sectional area, and an enlarged portion 72 provided on a downstream side of the linear portion 71 and extending in the Z direction such that the cross-sectional area increases as the enlarged portion 72 extends downstream.

The buffer space BF (refer to FIG. 8) that is a space into which air is introduced from outside by the intake unit 5 is provided on one side and the other side in the X direction of the duct 7 inside the housing 2. The buffer space BF is a space defined by inner surfaces of the housing 2 and outer surfaces of the linear portion 71 and the enlarged portion 72 of the duct 7. Lower end portions of the duct 7 are inserted and fixed to grooves 47 formed in the heat radiation fins 42 of the heatsink 4. An upper end portion of the duct 7 is fixed to a suction side of the fan 61. The duct 7 is attached to the housing 2 through the support frame 27.

As illustrated in FIGS. 2, 3, 4, and 5, the inert gas supply unit 8 supplies inert gas to the outside of the housing 2. Examples of the inert gas include nitrogen. The inert gas supply unit 8 forms a region, which is dominated by the inert gas (region with low oxygen concentration), in a region including an irradiation region of ultraviolet rays from the plurality of LED elements 32, by supplying the inert gas. The inert gas supply unit 8 is attached to a lower end portion of the wall portion 2c on the front side of the housing 2. The inert gas supply unit 8 includes a purge housing (housing) 81, a socket (connection portion) 82, and a throttle portion 88. The purge housing (housing) 81 is provided with a spray port 83. The purge housing 81 has, for example, a rectangular box shape. The second pipe Pa is connected to the socket 82. Namely, the socket 82 is a connection portion to which the second pipe Pa for supplying the inert gas into the purge housing 81 can be connected. In the inert gas supply unit 8, the inert gas is introduced from the second pipe Pa into the purge housing 81 through the socket 82 and the throttle portion 88, and the inert gas is sprayed from the spray port 83.

The inert gas suction unit 9 suctions the inert gas outside the housing 2, and causes the inert gas to flow into the housing 2. The inert gas suction unit 9 is a structure attached to the housing 2. The inert gas suction unit 9 is detachably attached to a rear side of the lower wall 2b of the housing 2 by fasteners such as screws. The inert gas suction unit 9 includes a suction unit housing 91 having a rectangular box shape; a suction port 92 provided in a lower surface of the suction unit housing 91; and a recovery flow path 93 provided inside the suction unit housing 91 (refer to FIG. 7). In the inert gas suction unit 9, the inert gas is suctioned into the suction unit housing 91 through the suction port 92, and the inert gas is allowed to flow through the recovery flow path 93 into the housing 2.

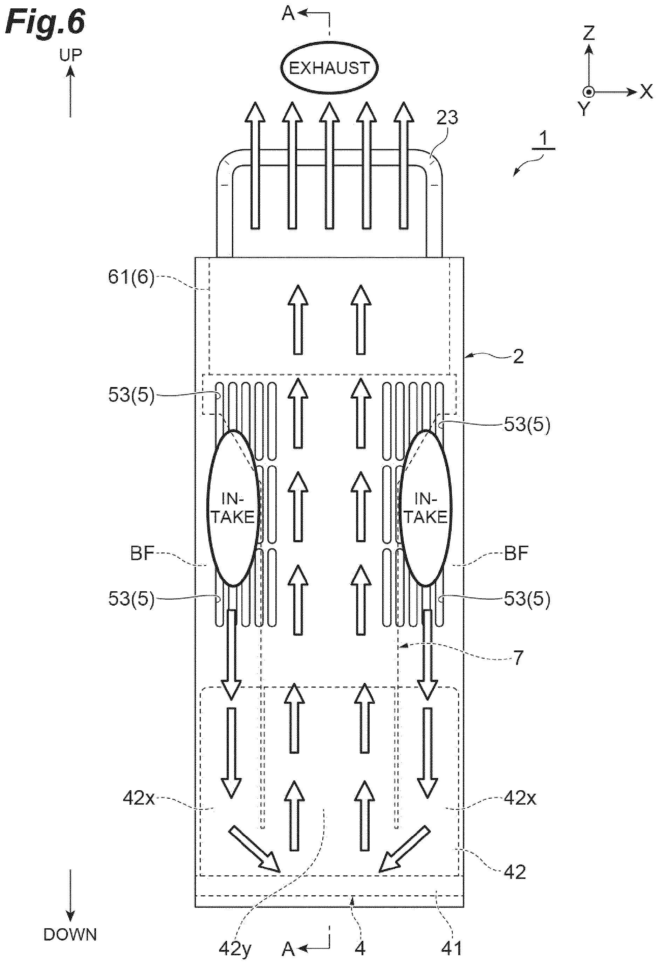

As illustrated in FIGS. 6, 7, and 8, in the active energy irradiation device 1, outside air is introduced into the buffer space BF inside the housing 2 by the intake unit 5. The air introduced into the buffer space BF flows downward along the Z direction, and then passes through the heatsink 4 and flows into the duct 7. At this time, in the heatsink 4, the air flows downward along the Z direction between the plurality of heat radiation fins 42 of each of the pair of outer portions 42x, and then flows to pass through between the partition plates 44 and the base plate 41 and to turn upward, and merges at the inner portion 42y. Then, the air flows upward along the Z direction between the plurality of heat radiation fins 42 of the inner portion 42y, and flows into the duct 7.

In addition, the air introduced into the buffer space BF flows downward along the Z direction, and then passes through the driver substrate heatsink 13. The air that has passed through the driver substrate heatsink 13 merges with the flow in the inner portion 42y of the heatsink 4 through a lower rear space inside the housing 2, flows upward along the Z direction between the plurality of heat radiation fins 42 of the inner portion 42y, and flows into the duct 7. The air that has flowed into the duct 7 flows upward along the Z direction, and is discharged to the outside of the housing 2 through the fan 61.

As illustrated in FIG. 7, in the active energy irradiation device 1, the inert gas sprayed from the inert gas supply unit 8 is suctioned by the inert gas suction unit 9, and flows into the housing 2. The inert gas that has flowed into the housing 2 merges with the flow in the inner portion 42y of the heatsink 4 through the lower rear space inside the housing 2, flows upward along the Z direction between the plurality of heat radiation fins 42 of the inner portion 42y, together with the air, and flows into the duct 7. The inert gas that has flowed into the duct 7 flows upward along the Z direction, together with the air, and is discharged to the outside of the housing 2 through the fan 61, together with the air.

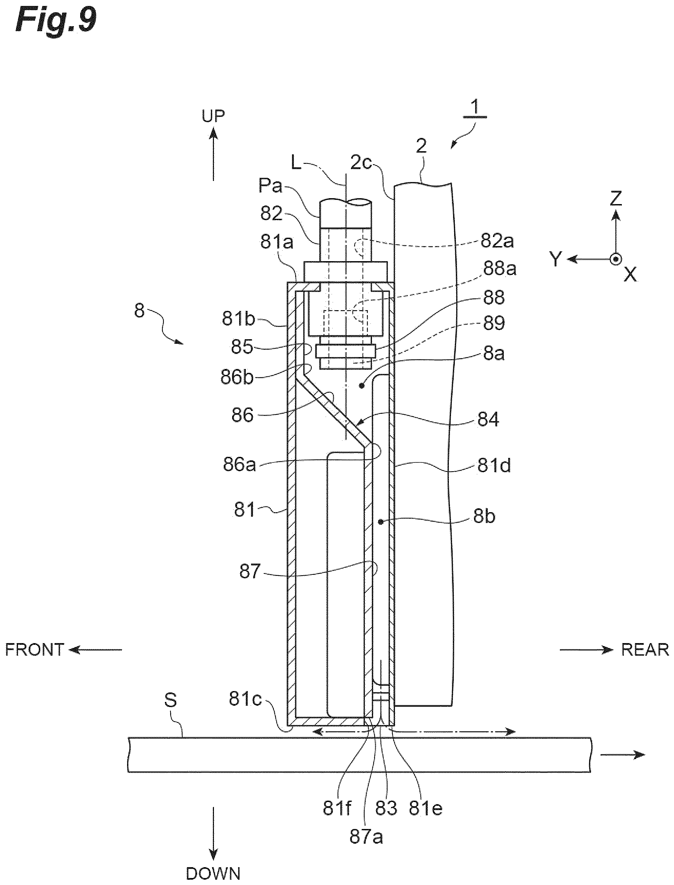

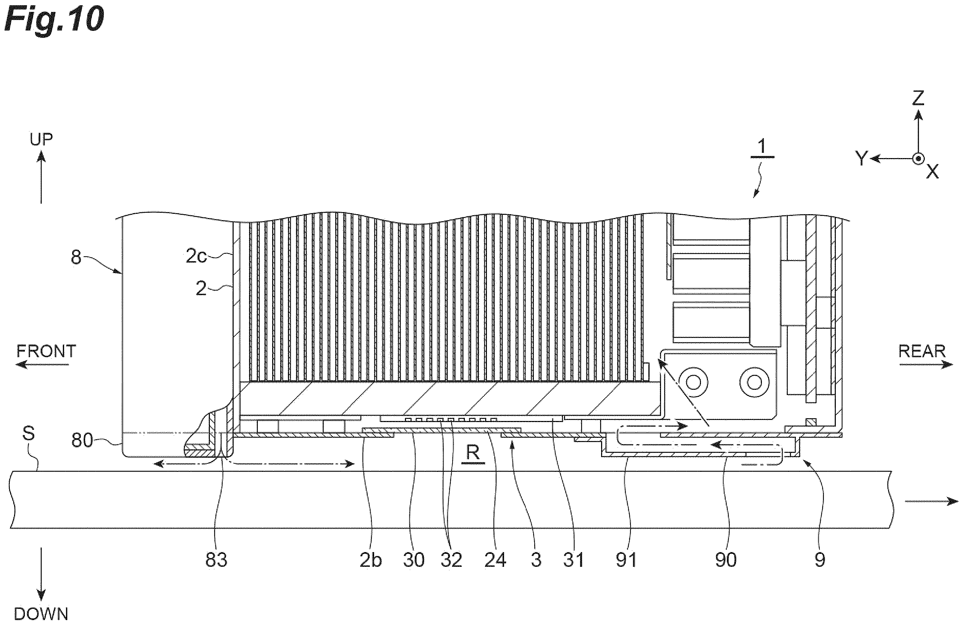

A configuration of the inert gas supply unit 8 will be described in more detail with reference to FIGS. 9 and 10.

As illustrated in FIGS. 9 and 10, the inert gas supply unit 8 supplies the inert gas to a region R between the active energy irradiation unit 3 and an irradiation target S conveyed from the front side to the rear side by a conveyor (not illustrated), by spraying the inert gas (alternate long and short dashed lines illustrated in FIGS. 9 and 10) onto the irradiation target S. The reason for supplying the inert gas to the region R is to prevent oxygen in the air from inhibiting an effect that occurs in the irradiation target S due to the irradiation with the ultraviolet rays emitted from the active energy irradiation unit 3.

As one example, the irradiation target S is a printed matter to which a photocurable ink is adhered. The ink is ejected onto the irradiation target S from an ink head (not illustrated) on an upstream side (front side) of the active energy irradiation device 1, and is cured by irradiation with ultraviolet rays emitted from the active energy irradiation unit 3. At this time, in order to prevent oxygen in the air from inhibiting the curing of the ink, the inert gas supply unit 8 supplies the inert gas to the region R between the irradiation target S and the active energy irradiation unit 3.

As illustrated in FIG. 9, the inert gas supply unit 8 includes a rectifying plate 84 inside the purge housing 81. The socket 82 and the throttle portion 88 are provided on a purge upper surface 81a of the purge housing 81. The socket 82 includes a flow path 82a for the inert gas. The throttle portion 88 includes a flow path 88a for the inert gas. The throttle portion 88 is provided on the socket 82 such that the flow path 82a and the flow path 88a communicate with each other. The throttle portion 88 is provided on the socket 82 so as to be located inside the purge housing 81. The throttle portion 88 is detachably attached to the socket 82. As one example, an upper end portion of the throttle portion 88 is attached to a lower end portion of the socket 82 by a fastening structure such as a screw. The throttle portion 88 includes a porous filter 89. The porous filter 89 is disposed in the flow path 88a such that a gap is formed between an inner surface of the flow path 88a and an outer surface of the porous filter 89. The porous filter 89 is made of a metal sintered body. The throttle portion 88 has a resistance causing the pressure of the inert gas within the second pipe Pa to be 0.1 MPa or higher.

The rectifying plate 84 is fixed to a purge front surface 81b of the purge housing 81. The rectifying plate 84 includes a first vertical surface 85, an inclined surface 86, and a second vertical surface 87. A purge bottom surface 81c of the purge housing 81 forms a part of the spray port 83. Namely, the spray port 83 is provided in the purge bottom surface 81c of the purge housing 81. A purge back surface 81d of the purge housing 81 is in contact with the front surface of the wall portion 2c of the housing 2. The rectifying plate 84 forms a flow path through which the inert gas passes, in cooperation with the purge housing 81.

The inert gas supply unit 8 includes a first flow path portion 8a and a second flow path portion 8b. The inert gas received from the socket 82 and the throttle portion 88 passes through the first flow path portion 8a, and then passes through the second flow path portion 8b, and is finally sprayed from the spray port 83.

The first flow path portion 8a receives the inert gas from the socket 82 and the throttle portion 88, and provides the inert gas to the second flow path portion 8b. The first flow path portion 8a is a region surrounded by the purge upper surface 81a, the first vertical surface 85, the inclined surface 86, and the purge back surface 81d. The first flow path portion 8a includes a portion that is reduced in flow path area along a direction in which the inert gas flows. Specifically, the first flow path portion 8a includes a portion between the first vertical surface 85 and the purge back surface 81d and a portion between the inclined surface 86 and the purge back surface 81d. The distance between the first vertical surface 85 and the purge back surface 81d is constant. Therefore, the flow path area is constant. On the other hand, the inclined surface 86 is inclined with respect to the Z direction. In other words, a lower side 86a of the inclined surface 86 is located behind an upper side 86b of the inclined surface 86 in the Y direction. Accordingly, the flow path area is gradually reduced as the inclined surface 86 approaches the second flow path portion 8b.

The socket 82 is provided on the purge upper surface 81a such that an axis L of the socket 82 and the throttle portion 88 is located substantially at the center between the purge front surface 81b and the purge back surface 81d. Namely, the axis L of the socket 82 and the throttle portion 88 intersects the inclined surface 86. In other words, the axis L of the socket 82 and the throttle portion 88 is located between the lower side 86a and the upper side 86b of the inclined surface 86 in the Y direction. Accordingly, the flow of the inert gas received from the socket 82 and the throttle portion 88 collides with the inclined surface 86. Then, the inert gas flows rearward in the Y direction along the inclined surface 86, and reaches the second flow path portion 8b.

The second flow path portion 8b receives the inert gas from the first flow path portion 8a, and provides the inert gas to the spray port 83. In other words, the second flow path portion 8b guides the inert gas to an irradiation target S side along the Z direction. The second flow path portion 8b is a region surrounded by the second vertical surface 87 and the purge back surface 81d. A region between an upper side of the second vertical surface 87 (namely, the lower side 86a of the inclined surface 86) and the purge back surface 81d receives the compressed inert gas. The distance between the second vertical surface 87 and the purge back surface 81d is constant. Namely, the flow path area of the second flow path portion 8b is constant. The flow path area of the second flow path portion 8b is smaller than a flow path area of a portion of the first flow path portion 8a between the first vertical surface 85 and the purge back surface 81d. The flow path area of the second flow path portion 8b is the same as a flow path area of a portion of the first flow path portion 8a between the lower side 86a of the inclined surface 86 and the purge back surface 81d.

A terminal of the second flow path portion 8b corresponds to the spray port 83. The spray port 83 is formed between a lower end 87a of the second vertical surface 87 and a lower end 81e of the purge back surface 81d. A rear end 81f of the purge bottom surface 81c may constitute a part of the spray port 83. The inert gas sprayed from the spray port 83 onto the irradiation target S collides with the irradiation target S, and then changes the flow direction. Some of the inert gas flows rearward and is supplied to the region R between the irradiation target S and the active energy irradiation unit 3 (refer to FIG. 10). Another portion of the inert gas flows forward and suppresses the flowing of air into the region R between the irradiation target S and the active energy irradiation unit 3 (refer to FIG. 10).

In the inert gas supply unit 8 configured as described above, the flow path area of the first flow path portion 8a is larger than the flow path area of each of the socket 82 and the second flow path portion 8b, and the rectifying plate 84 disposed to intersect the axis L of the socket 82 and the throttle portion 88 in the first flow path portion 8a hinders the flow of the inert gas received from the socket 82. Accordingly, the flow of the inert gas received from the socket 82 spreads out in the X direction, and as a result, the distribution of the flow rate of the inert gas sprayed from the spray port 83 is made uniform in the X direction. Therefore, the inert gas can be efficiently supplied to the region R between the irradiation target S and the active energy irradiation unit 3 (refer to FIG. 10). Further, the rectifying plate 84 has the inclined surface 86 as a surface that hinders the flow of the inert gas. Namely, the rectifying plate 84 realizes a flow path configuration in which the flow path area is gradually reduced. Accordingly, the operating noise of the inert gas supply unit 8 can be reduced.

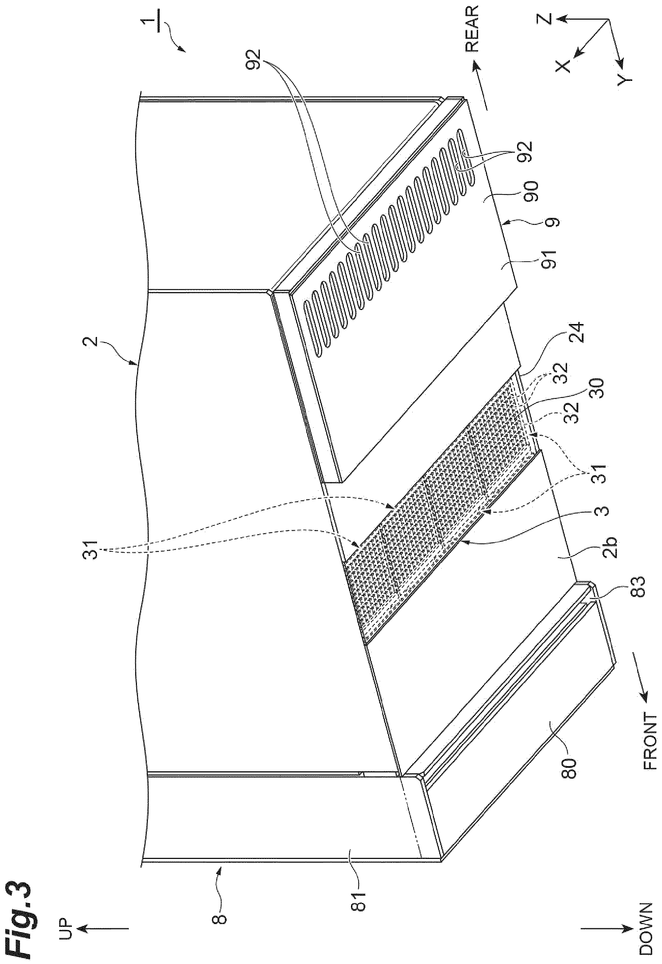

The housing 2, the active energy irradiation unit 3, the inert gas supply unit 8, and the inert gas suction unit 9 will be described in more detail with reference to FIGS. 3 and 10.

As illustrated in FIGS. 3 and 10, the active energy irradiation unit 3 has an emitting surface 30 that emits ultraviolet rays downward. In the active energy irradiation device 1, the emitting surface 30 is a surface on the lower side (outer surface) of the light irradiation window 24. The emitting surface 30 extends in both the X direction and the Y direction. In the active energy irradiation device 1, the emitting surface 30 is a surface perpendicular to the Z direction, and has a rectangular shape with the X direction as a longitudinal direction.

The inert gas supply unit 8 includes the spray port 83 that sprays the inert gas downward. The spray port 83 has an elongated shape with the X direction as a longitudinal direction. In the active energy irradiation device 1, the spray port 83 has a slit shape extending in the X direction. As one example, a width in the X direction of the spray port 83 is approximately 100 mm, and a width in the Y direction of the spray port 83 is approximately 5 mm. The spray port 83 is located in front of the emitting surface 30. The inert gas supply unit 8 includes a spray portion 80 provided with the spray port 83. The spray portion 80 is a portion of the inert gas supply unit 8, a portion protruding downward with respect to the emitting surface 30 (portion below an alternate long and two short dashed lines illustrated in FIGS. 3 and 10). In the active energy irradiation device 1, the outer shape of the spray portion 80 has a rectangular plate shape with the X direction as a longitudinal direction and the Z direction as a thickness direction. The inert gas supply unit 8 is attached to the housing 2 (specifically, the wall portion 2c on the front side) such that the spray portion 80 is located below the emitting surface 30 in the Z direction.

Incidentally, "the spray port 83 sprays the inert gas downward" means that the spray direction of the inert gas sprayed from the spray port 83 has a downward component. Namely, the spray port 83 may not only spray the inert gas directly downward, for example, but also spray the inert gas in a direction inclined rearward from directly below.

The inert gas suction unit 9 includes a protrusion portion 90 protruding downward with respect to the emitting surface 30. The protrusion portion 90 is located behind the emitting surface 30. In the active energy irradiation device 1, the entirety of the inert gas suction unit 9 is the protrusion portion 90. The inert gas suction unit 9 is attached to the housing 2 (specifically, the lower wall 2b) such that the protrusion portion 90 (namely, the entirety of the inert gas suction unit 9) is located below the emitting surface 30 in the Z direction. The inert gas suction unit 9 includes the suction unit housing 91 provided with a plurality of the suction ports 92. In the active energy irradiation device 1, the outer shape of the suction unit housing 91 has a rectangular plate shape with the X direction as a longitudinal direction and the Z direction as a thickness direction.

In the active energy irradiation device 1, the surface on the lower side (outer surface) of the lower wall 2b is exposed between the emitting surface 30 and the spray portion 80 and between the emitting surface 30 and the protrusion portion 90. A distance between the emitting surface 30 and the protrusion portion 90 in the Y direction is smaller than a distance between the emitting surface 30 and the spray portion 80 in the Y direction. The surface on the lower side of the lower wall 2b and a surface of the protrusion portion 90 are surfaces that absorb ultraviolet rays. As one example, the surface on the lower side of the lower wall 2b and the surface of the protrusion portion 90 may be black-painted surfaces, black-treated surfaces, or surfaces made of a black material.

A width in the Y direction of the protrusion portion 90 is larger than a width in the Z direction of the protrusion portion 90. The width in the Y direction of the protrusion portion 90 is larger than or equal to nine times the width in the Z direction of the protrusion portion 90. The width in the Y direction of the protrusion portion 90 is preferably larger than or equal to eight times the width in the Z direction of the protrusion portion 90, more preferably larger than or equal to ten times the width in the Z direction of the protrusion portion 90. A width in the Z direction of the spray portion 80 is larger than the width in the Z direction of the protrusion portion 90.

Each of a width in the X direction of the active energy irradiation unit 3, a width in the X direction of the inert gas supply unit 8, and a width in the X direction of the inert gas suction unit 9 is less than or equal to a width in the X direction of the housing 2. Each of the width in the X direction of the spray port 83 and a width in the X direction of the protrusion portion 90 is equal to a width in the X direction of the emitting surface 30. Here, being equal means substantially being equal, and for example, means that each of the width in the X direction of the spray port 83 and the width in the X direction of the protrusion portion 90 is 95% of the width in the X direction of the emitting surface 30 to 105% of the width in the X direction of the emitting surface 30. Incidentally, each of the width in the X direction of the spray port 83 and the width in the X direction of the protrusion portion 90 may be smaller than the width in the X direction of the emitting surface 30. Each of the width in the X direction of the spray port 83 and the width in the X direction of the protrusion portion 90 is preferably 90% or more, more preferably 100% or more, and further preferably 110% or more of the width in the X direction of the emitting surface 30.

As described above, in the active energy irradiation device 1, the socket 82 is provided on the purge housing 81 provided with the spray port 83, and the throttle portion 88 is provided on the socket 82. Accordingly, for example, even when the pressure of the inert gas fluctuates within the second pipe Pa connected to the socket 82, since the inert gas flowing from the second pipe Pa into the purge housing 81 through the socket 82 passes through the throttle portion 88, the flow rate of the inert gas supplied into the purge housing 81 is made uniform, and as a result, the flow rate of the inert gas sprayed from the spray port 83 is also made uniform. In addition, since the pressure of the inert gas decreases inside the purge housing 81, there is also no need to improve the pressure resistance and airtightness of the purge housing 81. Therefore, the active energy irradiation device 1 is less susceptible to structural constraints, and can uniformly spray the inert gas to the irradiation target S.

In the active energy irradiation device 1, the emitting surface 30 of the active energy irradiation unit 3 extends in both the X direction and the Y direction, and emits active energy rays downward, and the spray port 83 of the inert gas supply unit 8 is located in front of the emitting surface 30, and sprays the inert gas downward. Accordingly, when the irradiation target S is conveyed from the front side to the rear side with respect to the emitting surface 30 of the active energy irradiation unit 3, the inert gas sprayed from the spray port 83 of the inert gas supply unit 8 is supplied to the region R between the irradiation target S and the active energy irradiation unit 3. Therefore, the mixing of oxygen into the region R between the irradiation target S and the active energy irradiation unit 3 can be effectively suppressed.

In the active energy irradiation device 1, the spray port 83 has an elongated shape with the X direction as a longitudinal direction. Accordingly, the inert gas can be uniformly sprayed to the irradiation target S conveyed from the front side to the rear side with respect to the emitting surface 30 of the active energy irradiation unit 3.

In the active energy irradiation device 1, the throttle portion 88 includes the porous filter 89. In this case, with a simple structure, the flow rate of the inert gas supplied into the purge housing 81 can be made uniform.

In the active energy irradiation device 1, the porous filter 89 is made of a metal sintered body. In this case, with a simple structure, the flow rate of the inert gas supplied into the purge housing 81 can be made uniform.

In the active energy irradiation device 1, the throttle portion 88 is provided on the socket 82 so as to be located inside the purge housing 81. Accordingly, the flow rate of the inert gas supplied into the purge housing 81 can be made uniform.

In the active energy irradiation device 1, the throttle portion 88 has a resistance causing the pressure of the inert gas within the second pipe Pa to be 0.1 MPa or higher. Accordingly, the flow rate of the inert gas supplied into the purge housing 81 can be effectively made uniform.

In the active energy irradiation device 1, the throttle portion 88 is detachably attached to the socket 82. Accordingly, the replacement and maintenance of the throttle portion 88 can be easily performed.

In the active energy irradiation device 1, the active energy irradiation unit 3 emits ultraviolet rays as active energy rays. Accordingly, the active energy irradiation device 1 can be used as a device that irradiates the irradiation target S with ultraviolet rays.

In the active energy irradiation system 100, the flow rate of the inert gas sprayed from the inert gas supply unit 8 of each of the active energy irradiation devices 1 can be made uniform in the direction in which the plurality of active energy irradiation devices 1 are arranged (namely, the X direction).

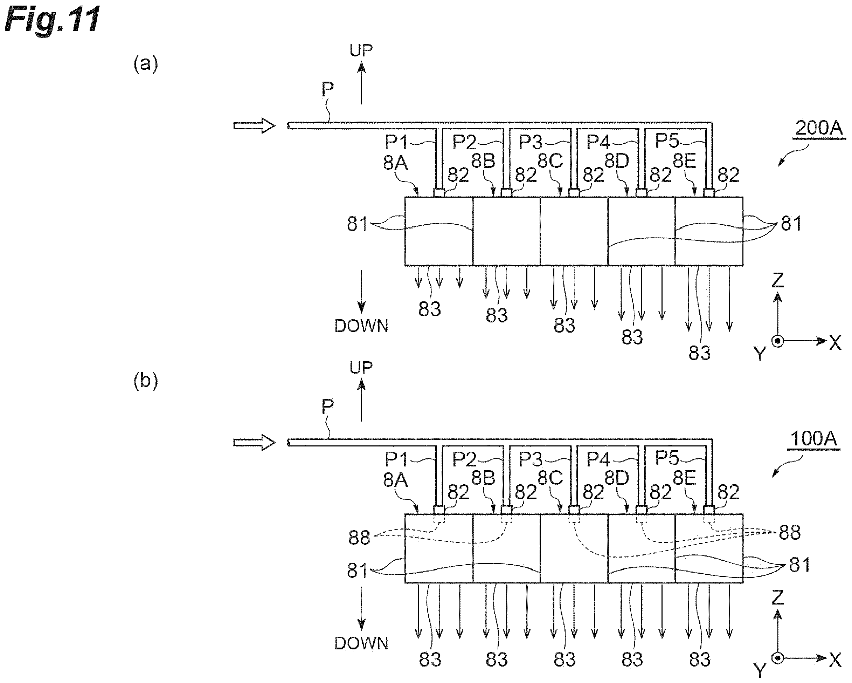

FIG. 11(a) is a front view of an active energy irradiation system 200A of a comparative example, and FIG. 11(b) is a front view of an active energy irradiation system 100A of an example. As illustrated in FIGS. 11(a) and 11(b), in each of the active energy irradiation systems 200A and 100A, a plurality of inert gas supply units 8A, 8B, 8C, 8D, and 8E are arranged in order from an upstream side of the first pipe P. A second pipe P1 is connected to the socket 82 of the inert gas supply unit 8A. A second pipe P2 is connected to the socket 82 of the inert gas supply unit 8B. A second pipe P3 is connected to the socket 82 of the inert gas supply unit 8C. A second pipe P4 is connected to the socket 82 of the inert gas supply unit 8D. A second pipe P5 is connected to the socket 82 of the inert gas supply unit 8E. Each of the second pipes P1, P2, P3, P4, and P5 corresponds to the second pipe Pa illustrated in FIGS. 1 and 9. A plurality of the second pipes P1, P2, P3, P4, and P5 branch off from the first pipe P in order from the upstream side of the first pipe P.

As illustrated in FIG. 11(a), in the active energy irradiation system 200A of the comparative example, the throttle portion 88 is not provided on the socket 82 in each of the inert gas supply units 8A, 8B, 8C, 8D, and 8E. As illustrated in FIG. 11(b), in the active energy irradiation system 100A of the example, the throttle portion 88 is provided on the socket 82 in each of the inert gas supply units 8A, 8B, 8C, 8D, and 8E. In the active energy irradiation system 100A of the example, the plurality of active energy irradiation devices 1 have the same configuration.

As illustrated in FIG. 11(a), in the active energy irradiation system 200A of the comparative example, the further downstream of the first pipe P the pipe branches off among the plurality of second pipes P1, P2, P3, P4, and P5, the higher the pressure of the inert gas becomes, so that the further downstream of the first pipe P the device is disposed among the plurality of inert gas supply units 8A, 8B, 8C, 8D, and 8E, the higher the flow rate of the inert gas sprayed from the spray port 83 becomes. On the other hand, as illustrated in FIG. 11(b), in the active energy irradiation system 100A of the example, the further downstream of the first pipe P the pipe branches off among the plurality of second pipes P1, P2, P3, P4, and P5, the higher the pressure of the inert gas becomes; however, due to the action of the throttle portion 88 described above, the flow rate of the inert gas sprayed from the spray port 83 of each of the inert gas supply units 8A, 8B, 8C, 8D, and 8E becomes uniform.

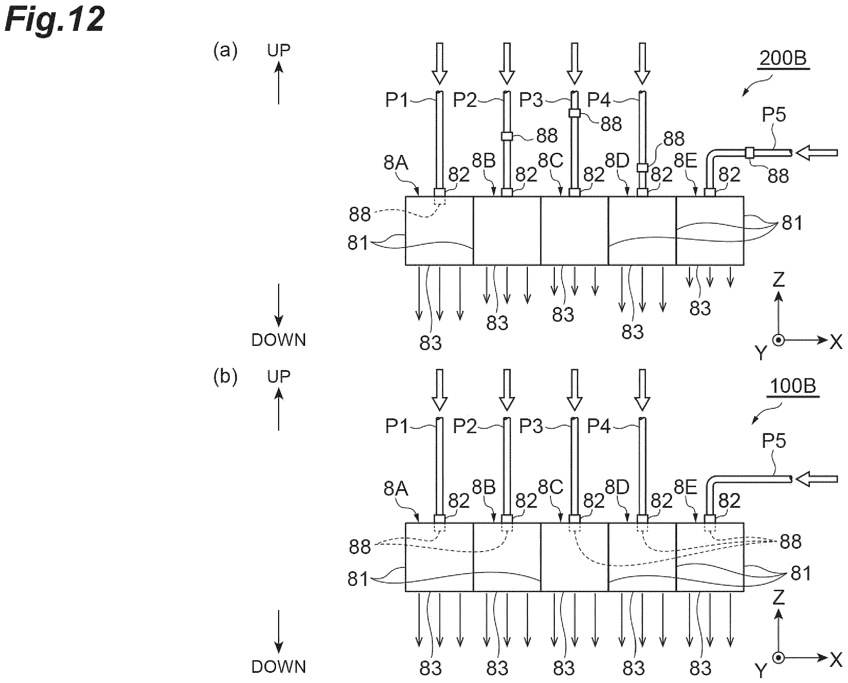

FIG. 12(a) is a front view of an active energy irradiation system 200B of a comparative example, and FIG. 12(b) is a front view of an active energy irradiation system 100B of an example. As illustrated in FIGS. 12(a) and 12(b), in each of the active energy irradiation systems 200B and 100B, the plurality of inert gas supply units 8A, 8B, 8C, 8D, and 8E are arranged in order. The second pipe P1 is connected to the socket 82 of the inert gas supply unit 8A. The second pipe P2 is connected to the socket 82 of the inert gas supply unit 8B. The second pipe P3 is connected to the socket 82 of the inert gas supply unit 8C. The second pipe P4 is connected to the socket 82 of the inert gas supply unit 8D. A second pipe P5 is connected to the socket 82 of the inert gas supply unit 8E. Each of the second pipes P1, P2, P3, P4, and P5 corresponds to the second pipe Pa illustrated in FIGS. 1 and 9. The inert gas is supplied to each of the second pipes P1, P2, P3, P4, and P5 at the same flow rate.

As illustrated in FIG. 12(a), in the active energy irradiation system 200B of the comparative example, the positions where the throttle portions 88 are provided in the inert gas supply units 8A, 8B, 8C, 8D, and 8E are different from each other. Specifically, in the inert gas supply unit 8A, the throttle portion 88 is provided on the socket 82. In each of the inert gas supply units 8B, 8C, 8D, and 8E, the throttle portion 88 is provided in the course of the second pipe Pa. The distance from the socket 82 to the throttle portion 88 is different for each of the inert gas supply units 8B, 8C, and 8D. The distance from the socket 82 to the throttle portion 88 is equal for each of the inert gas supply units 8C and 8E; however, in the inert gas supply unit 8E, the second pipe Pa is bent between the socket 82 and the throttle portion 88. As illustrated in FIG. 12(b), in the active energy irradiation system 100B of the example, the throttle portion 88 is provided on the socket 82 in each of the inert gas supply units 8A, 8B, 8C, 8D, and 8E. In the active energy irradiation system 100B of the example, the plurality of active energy irradiation devices 1 have the same configuration.

As illustrated in FIG. 12(a), in the active energy irradiation system 200B of the comparative example, the longer the distance from the socket 82 to the throttle portion 88 becomes in the plurality of inert gas supply units 8A, 8B, 8C, 8D, and 8E, the smaller the flow rate of the inert gas sprayed from the spray port 83 becomes. In the inert gas supply units 8C and 8E for each of which the distance from the socket 82 to the throttle portion 88 is equal, the flow rate of the inert gas sprayed from the spray port 83 becomes smaller in the inert gas supply unit 8E in which the second pipe Pa is bent between the socket 82 and the throttle portion 88. On the other hand, as illustrated in FIG. 12(b), in the active energy irradiation system 100B of the example, regardless of whether or not the second pipe Pa is bent, due to the action of the throttle portion 88 described above, the flow rate of the inert gas sprayed from the spray port 83 of each of the inert gas supply units 8A, 8B, 8C, 8D, and 8E becomes uniform.

The present disclosure is not limited to the above-described embodiment. For example, the socket 82 and the throttle portion 88 are not limited to being provided on the purge upper surface 81a, and may be provided at any position on the purge housing 81. The spray port 83 is not limited to being provided in the purge bottom surface 81c, and may be provided at any position in the purge housing 81. The throttle portion 88 may be provided on the socket 82 so as to be located outside the purge housing 81. Even in this case, the flow rate of the inert gas supplied into the purge housing 81 can be made uniform.

In the throttle portion 88, the porous filter 89 is not limited to being made of a metal sintered body, and may be made of ceramic, resin, or the like. The throttle portion 88 is not limited to including the porous filter 89, and may be a very small hole component, a throttle (aperture) made of such as metal or resin, or the like.

The active energy irradiation unit 3 is not limited to emitting ultraviolet rays, and may emit other active energy rays such as electron beams. Namely, in the above description, the "ultraviolet rays" can be reworded as "electron beams" or "active energy rays".

Various materials and shapes can be applied to each configuration in the embodiment and the modification examples described above without being limited to the materials and shapes described above. Each configuration in the embodiment or the modification examples described above can be arbitrarily applied to each configuration in other embodiments or modification examples.

### Reference Signs List

1: active energy irradiation device, 3: active energy irradiation unit, 30: emitting surface, 8: inert gas supply unit, 81: purge housing (housing), 82: socket (connection portion), 83: spray port, 88: throttle portion, 89: porous filter, 100: active energy irradiation system, P: first pipe, Pa: second pipe (pipe).

## Claims

1. An active energy irradiation device comprising:
an active energy irradiation unit having an emitting surface configured to emit an active energy ray; and
an inert gas supply unit having a spray port configured to spray an inert gas,
wherein the inert gas supply unit includes a housing provided with the spray port, a connection portion provided to the housing, and a throttle portion provided to the connection portion, and
a pipe for supplying the inert gas into the housing is connectable to the connection portion.

2. The active energy irradiation device according to claim 1,
wherein the emitting surface extends in both a first direction and a second direction perpendicular to the first direction, and is configured to emit the active energy ray to one side in a third direction perpendicular to both the first direction and the second direction, and
the spray port is located on one side in the second direction with respect to the emitting surface, and is configured to spray the inert gas to the one side in the third direction.

3. The active energy irradiation device according to claim 2,
wherein the spray port has an elongated shape with the first direction as a longitudinal direction.

4. The active energy irradiation device according to any one of claims 1 to 3,
wherein the throttle portion includes a porous filter.

5. The active energy irradiation device according to claim 4,
wherein the porous filter is made of a metal sintered body.

6. The active energy irradiation device according to any one of claims 1 to 5,
wherein the throttle portion is provided to the connection portion so as to be located inside the housing.

7. The active energy irradiation device according to any one of claims 1 to 5,
wherein the throttle portion is provided to the connection portion so as to be located outside the housing.

8. The active energy irradiation device according to any one of claims 1 to 7,
wherein the throttle portion has a resistance causing a pressure of the inert gas within the pipe to be 0.1 MPa or higher, the pipe connected to the connection portion.

9. The active energy irradiation device according to any one of claims 1 to 8,
wherein the throttle portion is detachably attached to the connection portion.

10. The active energy irradiation device according to any one of claims 1 to 9,
wherein the active energy irradiation unit emits an ultraviolet ray or an electron beam as the active energy ray.

11. An active energy irradiation system comprising:
a plurality of active energy irradiation devices each of which is the active energy irradiation device according to any one of claims 1 to 10;
a first pipe for supplying the inert gas into the housing of each of the plurality of active energy irradiation devices; and
a plurality of second pipes branching off from the first pipe, and connected to the connection portion of each of the plurality of active energy irradiation devices,
wherein the plurality of active energy irradiation devices are arranged in at least one row.
